# EUROPEAN PATENT APPLICATION

(11) **EP 3 308 792 A1**
(43) Date of publication of application: **18.04.2018**
(21) Application number: 16807632.1
(22) Date of filing: 10.06.2016
(51) Int. Cl.: A61K 33/06, A61K 9/10, A61K 33/08, A61K 47/02, A61K 47/18, A61K 47/22, A61K 47/26, A61K 47/32, A61K 47/34, A61K 47/36, A61K 47/38, A61K 47/40, A61K 47/42, A61P 17/02, A61P 17/04, A61P 37/08

(54) **CALCIUM SALT COMPOSITION AND FILAGGRIN PRODUCTION PROMOTER USING SAME**

(30) Priority: 12.06.2015 JP 2015119830
(71) Applicant: Nissan Chemical Industries, Ltd., Chiyoda-ku Tokyo 101-0054 (JP)
(72) Inventor: HIRATA, Osamu, Funabashi-shi Chiba 274-8507 (JP); IWAMA, Takehisa, Funabashi-shi Chiba 274-8507 (JP)
(74) Representative: dompatent von Kreisler Selting Werner - Partnerschaft von Patent- und Rechtsanwälten mbB
(86) International application number: PCT/JP2016/067454
(87) International publication number: WO 2016/199927

(57) **Abstract**

The objective of the present invention is to provide a calcium salt composition with improved dispersity and preservation stability of calcium salt in an aqueous medium as well as with increased filaggrin production promoting action of calcium salt in addition. The present invention relates to a calcium salt composition comprising a calcium salt insoluble or poorly soluble to an aqueous medium and an anionic dispersant.

## Description

### TECHNICAL FIELD

The present invention is related to a calcium salt composition with superior calcium salt dispersity and preservation stability as well as the use of the same as a filaggrin production promoter and an anti-aging agent.

### BACKGROUND ART

Filaggrin which is a constituent component of skin is involved in skin barrier function and is believed to be an important protein involved in skin function such as preventing the invasion of allergens, toxins, infectious organisms, and the like. Furthermore, in the recent years, its involvement in the risk of developing allergic diseases including atopic dermatitis has been revealed (see Non-Patent Document 1).

Furthermore, amino acid, urocanic acid, and the like, that are main components of natural moisturizing factors (NMF), are produced by the degradation of filaggrin in the stratum corneum. This filaggrin is known to be accumulated as profilaggrin in the epidermal keratinocytes present in the granular layer below the stratum corneum, degraded to filaggrin via dephosphorylation and hydrolysis, migrated to the stratum corneum, and thereby involved in the internal construction of corneocytes (see non-Patent Document 2).

From these results, increasing the filaggrin production of epidermal corneocytes is expected to essentially improve the moisture environment of the stratum corneum of human skin, maintain the skin moisture homeostasis, and furthermore, promote the improvement of chronic skin diseases such as atopic diseases.

Conventionally, licorice root extract (see Patent Document 1), heat-pressure processed rice koji (see Patent Document 2), and pharmaceutical screening compounds (see Patent Documents 3) are known as products having a profilaggrin production promoting action or filaggrin production promoting action.

### PRIOR ART DOCUMENTS

### PATENT DOCUMENTS

Patent Document 1: JP 2002-363054 A
Patent Document 2: JP 2014-240361 A
Patent Document 3: JP 2014-224109 A
Patent Document 4: JP 2012-77044 A
Patent Document 5: JP 3827259 B2

### NON-PATENT DOCUMENTS

Non-Patent Document 1: Journal of Kyoto Prefectural University of Medicine, 119, 877 (2010)
Non-Patent Document 2: Farumashia, 50, 973 (2014)
Non-Patent Document 3: The Journal of Investigative Dermatology, 81, 33s (1983)
Non-Patent Document 4: The Journal of Investigative Dermatology, 106, 254 (1996)

### SUMMARY OF INVENTION

### Problem to be Solved by the Invention

The present inventors have found an active ingredient having an anti-aging action or whitening action from highly safe materials and has carried out dedicated studies in the purpose of providing medicaments, quasi drugs, and cosmetics formulated with them.

Here, calcium ions are known to modify the proliferation or differentiation ability of human skin keratinocytes in a concentration-dependent manner (see Non-Patent Documents 3 and 4). While utilizing this specific action of calcium ion, it has been proposed that the calcium salt is used in the improvement of a turnover of human skin keratinocytes (Patent Document 4) and in the improvement of skin barrier as epidermal keratinization promoter (Patent Document 5), in cosmetics, medicaments, and quasi-drugs.

However, when considering the actual use of calcium ion in cosmetic and medical use, applying an appropriate amount of calcium ion to the skin at a predetermined concentration is difficult due to the issue of dryness and dripping. Furthermore, the calcium ion being washed out by sebum and sweat is also a problem.

Furthermore, as calcium ion is a divalent ion, it easily forms a chelate with a thickener, an active ingredient and the like formulated in cosmetics and the like, and thus, there are cases where a stable formulation may be difficult.

### Means for Solving the Problem

The present inventors have carried out dedicated research to find that when a calcium salt insoluble or poorly soluble to an aqueous medium is dispersed in a medium, it exerts a good filaggrin production promoting action, and that a calcium salt composition comprising an calcium salt insoluble or poorly soluble to an aqueous medium and an anionic dispersant improves the dispersity of calcium salt in an aqueous medium, and further enhances the filaggrin production promoting action thereof, and thereby completed the present invention.

That is to say that the present inventions are related to:
(1) a calcium salt composition comprising a calcium salt insoluble or poorly soluble to an aqueous medium and an anionic dispersant;
(2) the calcium salt composition according to the above-mentioned (1), further comprising an aqueous medium;
(3) the calcium salt composition according to the above-mentioned (1) or (2), wherein the weight ratio of the calcium salt and the dispersant is 1 : 0.5 to 1 : 100;
(4) the calcium salt composition according to any one of the above-mentioned (1) to (3), wherein the calcium salt is an organic salt or inorganic salt of calcium;
(5) the calcium salt composition according to the above-mentioned (4), wherein the organic salt of calcium is at least one selected from the group consisting of calcium glycerophosphate, calcium alginate, carboxymethylcellulose calcium, calcium citrate, calcium stearate, calcium myristate, and calcium 5'-ribonucleotides;
(6) the calcium salt composition according to the above-mentioned (4), wherein the inorganic salt of calcium is at least one selected from the group consisting of calcium hydrogen phosphate, calcium pyrophosphate, calcium dihydrogen pyrophosphate, tricalcium phosphate, calcium dihydrogen phosphate, calcium silicate, calcium carbonate, calcium oxide, calcium sulfate, and apatite;
(7) the calcium salt composition according to any one of the above-mentioned (1) to (6), wherein the anionic dispersant is at least one selected from the group consisting of ascorbic acid, tranexamic acid, water-soluble macromolecules, and phosphate derivatives;
(8) the calcium salt composition according to the above-mentioned (7), wherein the water-soluble macromolecule is at least one selected from the group consisting of gum arabic, tragacanth gum, galactan gum, guar gum, carrageenan, xanthan gum, deacylated gellan gum, maltose, trehalose, chitin, chitosan, agar, starch, dextrans, cyclodextrins, succinoglucan, collagen, casein, albumin, gelatin, hyaluronic acid and its salts, chondroitin sulfate, carboxymethylcellulose, methylhydroxypropyl starch, methylcellulose, pullulan, pectin, nitrocellulose, ethyl cellulose, methylhydroxypropyl cellulose, hydroxyethyl cellulose, sodium cellulose sulfate, hydroxypropyl cellulose, sodium carboxymethylcellulose, microcrystalline cellulose, sodium alginate, propylene glycol alginate ester, polyvinyl methyl ether, carboxyvinyl polymers, polyvinyl alcohol, polyvinyl pyrrolidone, polyoxyethylene polyoxypropylene copolymer macromolecules, acrylicpolymers, polyethylene imine, cationized guar gum, and cationized cellulose;
(9) the calcium salt composition according to the above-mentioned (7), wherein the phosphate derivative is at least one selected from the group consisting of phosphate, hydrogen phosphate, polyphosphate, metaphosphate, L-ascorbate-phosphate, adenosine-phosphate, dialkyl phosphate, monoalkyl phosphate, dipolyoxyethylene phosphate, monopolyoxyethylene phosphate, and phytate;
(10) the calcium salt composition according to any one of the above-mentioned (1) to (9) comprising at least two kinds of the anionic dispersant;
(11) a filaggrin production promoter comprising the calcium salt composition according to any one of the above-mentioned (1) to (10) or a calcium salt insoluble or poorly soluble to an aqueous medium;
(12) an anti-aging agent comprising the calcium salt composition according to any one of the above-mentioned (1) to (10) or a calcium salt insoluble or poorly soluble to an aqueous medium;
(13) a filaggrin production promoter comprising a calcium salt insoluble or poorly soluble to an aqueous medium, which is ground by dry grinding;
(14) a filaggrin production promoter comprising a calcium salt composition in which a calcium salt insoluble or poorly soluble to an aqueous medium and an anionic dispersant are dry ground together;
(15) the filaggrin production promoter according to the above-mentioned (13) comprising the calcium salt and an aqueous medium;
(16) the filaggrin production promoter according to the above-mentioned (14) comprising the calcium salt composition and an aqueous medium;
(17) a method for producing a filaggrin production promoter comprising a step of dry grinding a calcium salt insoluble or poorly soluble to an aqueous medium;
(18) the method for producing according to the above-mentioned (17) further comprising a step of dispersing in an aqueous medium subsequent to the step of dry grinding;
(19) a method for producing a filaggrin production promoter comprising a step of dry grinding a calcium salt insoluble or poorly soluble to an aqueous medium and an anionic dispersant together;
(20) the method for producing according to the above-mentioned (19) further comprising a step of dispersing in an aqueous medium subsequent to the step of dry grinding;
(21) a method for promoting filaggrin production using the calcium salt composition according to any one of the above-mentioned (1) to (10) or a calcium salt insoluble or poorly soluble to an aqueous medium, and
(22) a method for regulating the particle size of calcium salt characterized in that two or more kinds of the anionic dispersant are mixed with the calcium salt.

### EFFECTS OF THE INVENTION

A calcium salt insoluble or poorly soluble to an aqueous medium of the present invention promotes the production of filaggrin and can exert an effect as an anti-aging agent both having a superior anti-aging action and safeness.

Moreover, according to the present invention, a calcium salt composition with superior dispersity and preservation stability of calcium salt in a medium can be obtained. Accordingly, such calcium salt composition could improve the filaggrin production promoting action of calcium salt and can exert an effect as an anti-aging agent both having a superior anti-aging action and safeness.

### BRIEF DESCRIPTION OF THE DRAWINGS

FIG.1 is a diagram showing the distribution of particle size per scattering intensity of DLS of Example 6.
FIG.2 is a diagram showing the distribution of particle size per individual number of DLS of Example 6.
FIG. 3 is a fluorescent microscopic image of Comparative Test Example 7.
FIG. 4 is a fluorescent microscopic image of Comparative Test Example 8.
FIG. 5 is a fluorescent microscopic image of Comparative Test Example 9.
FIG. 6 is a fluorescent microscopic image of Comparative Test Example 10.
FIG. 7 is a fluorescent microscopic image of Test Example 20.
FIG. 8 is a diagram showing the dot plot results of Test Example 21 and Comparative Test Examples 11 and 12.

### Mode for Carrying out the Invention

In the present invention, a calcium salt insoluble or poorly soluble to an aqueous medium is referred to a calcium salt whose solubility at 20°C to the aqueous medium is lower than that of the calcium chloride.

Here, as defined in the Handbook of Chemistry (Revised version III, MARUZEN PUBLISHING CO. Ltd., 1991, Tokyo), solubility at 20°C to the aqueous medium refers to an amount of calcium salt (g) dissolved in 100 g of a saturated solution which was obtained by saturating an aqueous medium with calcium salt at 20°C. For example, in case of water being the aqueous medium, the solubility of calcium chloride in pure water at 20°C is 42.7, and thus, a calcium salt insoluble or poorly soluble to water refers to a calcium salt whose solubility in water at 20°C is less than 42.7.

An aqueous medium of the present invention refers to water, phosphate buffered saline (PBS), hydrophilic medium, or a mixture of two more media selected from these.

Moreover, the above-mentioned hydrophilic medium is not particular limited so long as it is conventionally used in the field of medicaments or cosmetics, and it is not particularly limited as long as it is in a form of liquid at ordinary temperature with superior compatibility with water. For example, alcohol is preferred, and specifically, monovalent lower alcohol such as ethanol, n-propanol, isopropanol, and n-butanol; diavalent alcohol such as 1,3-butylene glycol, ethylene glycol, and propylene glycol; polyalkylene glycols such as polyethylene glycol, dipropylene glycol, and polypropylene glycol; and polyvalent alcohols such as glycerin, diglycerin, trimethylolpropane, pentaerythritol, and sorbitol are exemplified. Among these, ethanol or glycerin is particularly preferred. These may be used alone or two more may be used in combination.

Among the above-mentioned aqueous media, because calcium salt is well-dispersed, water, phosphate buffered saline (PBS), a mixture of water and ethanol, and a mixture of water and glycerine are preferred, water as well as a mixture of water and glycerine is more preferred, and water is most preferred.

When the above-mentioned aqueous medium is water or a mixture of PBS with a hydrophilic medium, for example, to 100 weights by part of a hydrophilic medium, water or PBS is contained at 0.5 to 100 parts by weight, preferably 30 to 100 parts by weight, and more preferably 80 to 100 parts by weight.

In the present invention, a calcium salt insoluble or poorly soluble to an aqueous medium is preferably ground by dry grinding from the point of view that the calcium salt has the good dispersity and preservation stability in a medium such as water, and promotes filaggrin production.

In the present invention, a calcium salt insoluble or poorly soluble to an aqueous medium is preferred to have a particle size of several dozens to several hundreds of nm from the point of view that it has the good dispersity and preservation stability in a medium such as water, and promotes filaggrin production. For example, it is preferred that the average particle size of scattering intensity measured by DLS is 50 nm to 300 nm, and in particular 100 nm to 300 nm.

Furthermore, the above-mentioned calcium salt insoluble or poorly soluble to an aqueous medium is preferred to be dispersed as a negatively charged particle in a medium such as water from the point of view that it has the good dispersity and preservation stability in a medium such as water, and promotes filaggrin production. For example, it is preferred that the zeta potential is -60mV to -30mV.

The calcium salt composition of the present invention comprises a calcium salt insoluble or poorly soluble to the above-mentioned aqueous medium and an anionic dispersant.

The filaggrin production promoter of the present invention comprises a calcium salt insoluble or poorly soluble to the above-mentioned aqueous medium, and more preferably further comprises an anionic dispersant.

The calcium salt composition of the present invention may further comprise the aqueous medium defined above in addition to the above-mentioned calcium salt and anionic dispersant. The kind and amount of this aqueous medium is not particularly limited as long as the calcium salt is dispersed in the obtained calcium salt composition.

The weight ratio of the calcium salt and dispersant in the calcium salt composition of the present invention is not particularly limited as long as the condition allows the dispersion of the calcium salt to the medium; however, preferably, it is 1 : 0.5 to 1 : 100, more preferably 1 : 4 to 1 : 100, or 1 : 1 to 1 : 20, and more preferably, 1 : 5 to 1 : 20. In particular, as the particle size of the calcium salt becomes smaller in the calcium salt composition, it is preferred to increase the amount of the dispersant. Nonetheless, the maximum amount of the dispersant is 100 to 1 of calcium salt, by weight ratio, for example.

The calcium salt of the present invention is not particularly limited so long as it is a calcium salt insoluble or poorly soluble to an aqueous medium. For example, it may be either an organic salt or inorganic salt of calcium.

As the above-mentioned organic salts of calcium, calcium glycerophosphate, calcium alginate, carboxymethylcellulose calcium, calcium citrate, calcium stearate, calcium myristate and calcium 5'-ribonucleotides are exemplified, and calcium glycerophosphate, calcium citrate, calcium alginate and calcium 5'-ribonucleotides are preferred, and calcium glycerophosphate is more preferred.

Further, as the above-mentioned inorganic salts of calcium, calcium hydrogen phosphate, calcium pyrophosphate, calcium dihydrogen pyrophosphate, tricalcium phosphate, calcium dihydrogen phosphate, calcium silicate, calcium carbonate, calcium oxide, calcium sulfate and apatite are exemplified, and calcium hydrogen phosphate, calcium pyrophosphate, tricalcium phosphate, calcium dihydrogen phosphate, apatite, calcium silicate, calcium carbonate and calcium sulfate are preferred, calcium hydrogen phosphate, calcium pyrophosphate, tricalcium phosphate, calcium dihydrogen phosphate, apatite, calcium silicate and calcium carbonate are more preferred, calcium hydrogen phosphate, calcium pyrophosphate, tricalcium phosphate, calcium dihydrogen phosphate and apatite are further preferred, and tricalcium phosphate is most preferred.

As for the anionic dispersant of the present invention, ascorbic acid (e.g., L-ascorbic acid), tranexamic acid, water-soluble macromolecule and phosphate derivatives are exemplified, and water-soluble macromolecule and phosphate derivatives are preferred.

As the above-mentioned water-soluble macromolecules, gum arabic, tragacanth gum, galactan gum, guar gum, carrageenan (e.g., κ-carrageenan), xanthan gum, deacylated gellan gum, maltose, trehalose, chitin, chitosan, agar, starch, dextrans, cyclodextrins (for example, α-cyclodextrin, γ-cyclodextrin, and branched cyclodextrins), succinoglucan, collagen, casein, albumin, gelatin, hyaluronic acid and its salts (e.g., sodium hyaluronate), chondroitin sulfate, carboxymethylcellulose, methylhydroxypropyl starch, methylcellulose, pullulan, pectin, nitrocellulose, ethyl cellulose, methylhydroxypropyl cellulose, hydroxyethyl cellulose, sodium cellulose sulfate, hydroxypropyl cellulose, sodium carboxymethylcellulose, microcrystalline cellulose, sodium alginate, propylene glycol alginate ester, polyvinyl methyl ether, carboxyvinyl polymers, polyoxyethylene polyoxypropylene copolymer macromolecules, acrylic polymers (e.g., sodium polyacrylate, polyethyl acrylate, polyacrylamide), polyvinyl alcohol, polyvinyl pyrrolidone, polyethylene imine, cationized guar gum and cationized cellulose are exemplified, and deacylated gellan gum, sodium alginate, methyl cellulose, collagen, sodium hyaluronate, κ-carrageenan, maltose and trehalose are preferred, deacylated gellan gum, collagen, sodium hyaluronate, sodium alginate, methylcellulose and κ-carrageenan are more preferred, and collagen, sodium hyaluronate and sodium alginate are particularly preferred.

As the above-mentioned phosphate derivatives, phosphate (e.g., sodium phosphate), hydrogen phosphate (e.g., sodium hydrogen phosphate), polyphosphate (e.g., sodium polyphosphate), metaphosphate, L-ascorbate-phosphate (for example, L-ascorbic acid 2-phosphate sodium salt and L-ascorbic acid 2-phosphate magnesium salt), adenosine-phosphate (for example, adenosine 5-monophosphate sodium salt), dialkyl phosphate, monoalkyl phosphate, dipolyoxyethylene phosphate, monopolyoxyethylene phosphate, and phytate (e.g., sodium phytate) are exemplified, and adenosine-sodium phosphate, sodium phytate, sodium phosphate, sodium hydrogen phosphate, sodium polyphosphate, L-Ascorbic Acid-2-Phosphate Sodium and L-Ascorbic Acid-2-Phosphate Magnesium are preferred, sodium polyphosphate, L-Ascorbic Acid-2-Phosphate Sodium and L-Ascorbic Acid-2-Phosphate Magnesium are more preferred, and sodium polyphosphate is most preferred.

In the present invention, an anionic dispersant may be used alone or two or more kinds may be used in combination; however, from the point of view that a calcium salt particle with a particle size of 100 nm or less is obtained, it is preferred to use two or more, in particular two kinds of anionic dispersants, in combination. For example, it is preferred to use in combination two kinds of derivatives from phosphate derivatives, and the combined use of sodium polyphosphate and L-Ascorbic Acid-2-Phosphate Sodium is particularly preferred.

Among those mentioned above, a particularly preferred combination of a calcium salt and a dispersant is a combination of the calcium salt and dispersant disclosed in the Examples. For example, the combinations shown in Table 1 are provided.

**[Table 1]**

| Calcium salt | Dispersant |
|---|---|
| Tricalcium phosphate | Methylcellulose |
| Tricalcium phosphate | Deacylated gellan gum |
| Tricalcium phosphate | Sodium alginate |
| Tricalcium phosphate | κ-carrageenan |
| Tricalcium phosphate | Sodium hyaluronate |
| Tricalcium phosphate | Sodium polyphosphate |
| Tricalcium phosphate | L-ascorbic acid 2-phosphate sodium |
| Tricalcium phosphate | L-ascorbic acid 2-phosphate magnesium |
| Tricalcium phosphate | Ascorbic acid |
| Tricalcium phosphate | Tranexamic acid |
| Tricalcium phosphate, particle size 30 nm | Sodium polyphosphate |
| Apatite, particle size 40 nm | Sodium polyphosphate |
| Apatite, particle size 150 nm | Sodium polyphosphate |
| Apatite, particle size 5 µm | Sodium polyphosphate |
| Calcium dihydrogen phosphate | Sodium polyphosphate |
| Calcium glycerophosphate | Sodium polyphosphate |
| Tricalcium phosphate | Sodium polyphosphate, L-Ascorbic Acid-2-Phosphate Sodium |

The calcium salt composition of the present invention may be produced by conventional methods. However, the calcium salt composition is produced after a calcium salt is ground by dry grinding, more preferably, the calcium salt composition is produced by grinding a calcium salt by dry grinding and then mixing it with an anionic dispersant, and most preferably, the calcium salt composition is produced by grinding a calcium salt and an anionic dispersant together by dry grinding, from the point of view that the calcium salt has the good dispersity and preservation stability in a medium such as water, and promotes the filaggrin production.

Furthermore, the calcium salt composition of the present invention may be obtained by grinding a calcium salt and an anionic dispersant by drying grinding and then further dispersing them in an aqueous medium.

As a condition of the above-mentioned dry grinding, it is not particularly limited as long as the condition allows the grinding of the calcium salt and anionic dispersant into small pieces; however, using a mil such as mortar, ball mill, and homogenizer is preferred. Furthermore, the grinding time, pressure applied, and the like, are appropriately modified depending on the hardness of the calcium salt and the dispersant to be ground.

From the point of view that a calcium salt in the obtained composition has the good dispersity and preservation stability and that the filaggrin production is promoted, it is preferred that dry grinding is performed using a ball mill (for example, manufactured by Retsch GmbH, MM200) at a frequency of 25Hz with 90-mintues treating time, or using a ball mill (for example, manufactured by Fritsch Co. Ltd, a planetary ball mill P-6) at 500 rpm.

In the present invention, by mixing two or more kinds of the above-mentioned anionic dispersants with the above-mentioned calcium salt, the particle size of the above-mentioned calcium salt can be regulated. This mixture can be carried out by conventional means; however, it is preferred to be carried out by the aforementioned dry grinding.

For example, due to the above-mentioned method, calcium salt particles of a particle size of 100 nm or less are obtained.

The calcium salt composition of the present invention or a calcium salt insoluble or poorly soluble to an aqueous medium can be used, for example, as a medicament, quasi drug, or cosmetics, for example, as a medicament for preventing and/or treating skin diseases, for example, allergic skin diseases such as atopic dermatitis, contact dermatitis and urticaria; a filaggrin production promoter; an anti-aging agent; a whitening agent; or the like.

To the calcium salt composition of the present invention, any active ingredient, for example, other component having anti-aging action or whitening action, may be added, as long as the effect of the present invention is not impaired.

The calcium salt composition of the present invention can contain excipients, sweeteners, acidulants, thickeners, fragrances, dyes and emulsifiers, as well as other component that can be generally formulated to medicaments, quasi drugs and cosmetics, as long as the effect of the present invention is not impaired. For examples, as such components, the followings are exemplified: polyvalent alcohols such as glycerin and propylene glycol, oils such as liquid paraffin, squalane, higher fatty acids and higher alcohols, organic acids such as citric acid and lactic acid, alkalis such as sodium hydroxide and triethanolamine, cationic surfactants, amphoteric surfactants, non-ionic surfactants, powders, pigments, dyes, preservative and fungicide, resin, pH adjusting agents, antioxidants, ultraviolet absorbers, chelating agents, thickeners, moisturizers, alcohols, water, perfume and the like.

In the present invention, an appropriate and effective amount of a calcium salt composition or a calcium salt insoluble or poorly soluble to an aqueous medium of the present invention can be administered to a subject such as a patient, for preventing and/or treating skin diseases, for example, allergic skin diseases such as atopic dermatitis, contact dermatitis and urticarial, or the like, for anti-aging agent or whitening, or for promoting filaggrin production. The effective amount and the intake period can be optionally defined in accordance to the age and symptoms of the subject.

### EXAMPLES

Hereinafter, the present invention will be described in details with reference to the Examples, but the present invention is not limited to the following Examples. Furthermore, the device, cells, and culture media used in the Examples are as follows:
- Ball mill; manufactured by Retsch GmbH, MM200
- Ball mill; manufactured by Fritsch Co. Ltd., Planetary ball mill P-6
- Dynamic light scattering apparatus (DLS); manufactured by Malvern Instruments Ltd, Zetasizer Nano ZS
- Centrifuge; manufactured by KUBOTA Corporation, Tabletop Centrifuge 4000
- Autoclave; manufactured by Tomy Seiko Co., Ltd, ES-215
- Incubator; manufactured by Panasonic Corp., KM-CC17RU2
- Fluorescence microscope; manufactured by KEYENCE Corporation, BIOREVO BZ-9000
- Medium; manufactured by KURABO INDUSTRIES LTD., HuMedia-KG2
- Cells; manufactured by KURABO INDUSTRIES LTD., NHEK (AD)

### 1. Preparation of calcium salt composition

### (1) Preparation Example 1 (Dispersity test)

Each calcium salt and dispersant was weighed according to Table 2, which were then subjected to a dry grinding and mixing process (25Hz, 90 minutes) together in a ball mill (manufactured by Retsch GmbH, MM200), and calcium salt compositions of Examples 1 to 30 were obtained. Note that in Examples 27 to 30, 2 kinds of dispersants were added to convert them into a ternary system.

**[Table 2]**

| | Composition | |
|---|---|---|
| | Calcium salt | Dispersant |
| Example 1 | Tricalcium phosphate (50) | Methylcellulose (450) |
| Example 2 | Tricalcium phosphate (50) | Deacylated gellan gum (450) |
| Example 3 | Tricalcium phosphate (50) | Sodium alginate (450) |
| Example 4 | Tricalcium phosphate (50) | κ-carrageenan (450) |
| Example 5 | Tricalcium phosphate (50) | Sodium hyaluronic acid (450) |
| Example 6 | Tricalcium phosphate (50) | Sodium polyphosphate (450) |
| Example 7 | Tricalcium phosphate (50) | L-Ascorbic Acid-2-Phosphate Sodium (450) |
| Example 8 | Tricalcium phosphate (50) | L-Ascorbic Acid-2-Phosphate Magnesium (450) |
| Example 9 | Tricalcium phosphate (50) | Ascorbic acid (450) |
| Example 10 | Tricalcium phosphate (50) | Tranexamic acid (450) |
| Example 11 | Tricalcium phosphate, particle size 30 nm (50) | Sodium polyphosphate (450) |
| Example 12 | Apatite, particle size 40 nm (50) | Sodium polyphosphate (450) |
| Example 13 | Apatite, particle size 150 nm (50) | Sodium polyphosphate (450) |
| Example 14 | Apatite, particle size 5 µm (50) | Sodium polyphosphate (450) |
| Example 15 | Calcium dihydrogen phosphate (50) | Sodium polyphosphate (450) |
| Example 16 | Calcium glycerophosphate (50) | Sodium polyphosphate (450) |
| Example 17 | Tricalcium phosphate (400) | Sodium polyphosphate (100) |
| Example 18 | Tricalcium phosphate (250) | Sodium polyphosphate (250) |
| Example 19 | Tricalcium phosphate (100) | Sodium polyphosphate (400) |
| Example 20 | Tricalcium phosphate (25) | Sodium polyphosphate (475) |
| Example 21 | Tricalcium phosphate (400) | Sodium alginate (100) |
| Example 22 | Tricalcium phosphate (250) | Sodium alginate (250) |
| Example 23 | Tricalcium phosphate (100) | Sodium alginate (400) |
| Example 24 | Tricalcium phosphate (25) | Sodium alginate (475) |
| Example 25 | Tricalcium phosphate (250) | L-Ascorbic Acid-2-Phosphate Sodium (250) |
| Example 26 | Tricalcium phosphate (100) | L-Ascorbic Acid-2-Phosphate Sodium (400) |
| Example 27 | Tricalcium phosphate (50) | Sodium polyphosphate (425), L-Ascorbic Acid-2-Phosphate Sodium (25) |
| Example 28 | Tricalcium phosphate (50) | Sodium polyphosphate (400), L-Ascorbic Acid-2-Phosphate Sodium (50) |
| Example 29 | Tricalcium phosphate (50) | Sodium polyphosphate (350), L-Ascorbic Acid-2-Phosphate Sodium (100) |
| Example 30 | Tricalcium phosphate (50) | Sodium polyphosphate (200), L-Ascorbic Acid-2-Phosphate Sodium (250) |

| | | |
|---|---|---|
| The numerical values inside the parentheses indicate the amount weighed (mg). | | |

### (2) Preparation Example 2 (Dispersity test, grinding time dependency)

Tricalcium phosphate/L-Ascorbic Acid-2-Phosphate Sodium (=1/9 (wt/wt)) mixed powder (3.0 g) was weighed, and this was subjected to a dry grinding and mixing process (500 rpm) in a ball mil (manufactured by Fritsch Co. Ltd., a planetary ball mill P-6). In doing so, grinding was stopped after 0.5, 1.0, 3.0, 5.0, 7.0 hours and 200 mg of the sample was sampled at every point.

### (3) Preparation Example 3

Without mixing a dispersant, 500 mg of only tricalcium phosphate was weighed, and this was subjected to a dry grinding and mixing process (25Hz, 90 minutes) in a ball mill to obtain a calcium salt powder of Preparation Example 3.

### 2. Dispersity test (A)

### (1) Dispersity test (water or phosphate buffered saline (PBS))

Some of the calcium salt compositions obtained in preparation Example 1 were dispersed in a dispersion medium [water or a phosphate buffered saline (PBS)] so that each of them would have a calcium salt concentration of 1.0 mg/2mL, and thereby a dispersed solution was prepared. The obtained dispersed solution was allowed to stand at room temperature for 1 hour after preparation and was observed. A dispersed solution in which a precipitation was not confirmed by visual inspection was assessed as "○" and a dispersed solution in which a precipitation was confirmed by visual inspection was assessed as "×". The results were shown in Table 3.

**[Table 3]**

| | Dispersion medium | |
|---|---|---|
| | Water | PBS |
| Example 1 | ○ | ○ |
| Example 3 | ○ | ○ |
| Example 5 | ○ | ○ |
| Example 6 | ○ | ○ |
| Example 7 | ○ | ○ |
| Example 8 | ○ | ○ |
| Example 9 | ○ | ○ |
| Example 11 | ○ | ○ |
| Example 12 | ○ | ○ |
| Example 13 | ○ | ○ |
| Example 14 | ○ | ○ |
| Example 15 | ○ | ○ |
| Example 16 | ○ | ○ |
| Example 19 | ○ | ○ |
| Example 20 | ○ | ○ |
| Example 23 | ○ | ○ |
| Example 24 | ○ | ○ |
| Example 25 | ○ | - |
| Example 26 | ○ | - |
| Example 27 | ○ | - |
| Example 28 | ○ | - |
| Example 29 | ○ | - |
| Example 30 | ○ | - |

| | | |
|---|---|---|
| -; Not performed | | |

### (2) Measurement of particle size and zeta potential

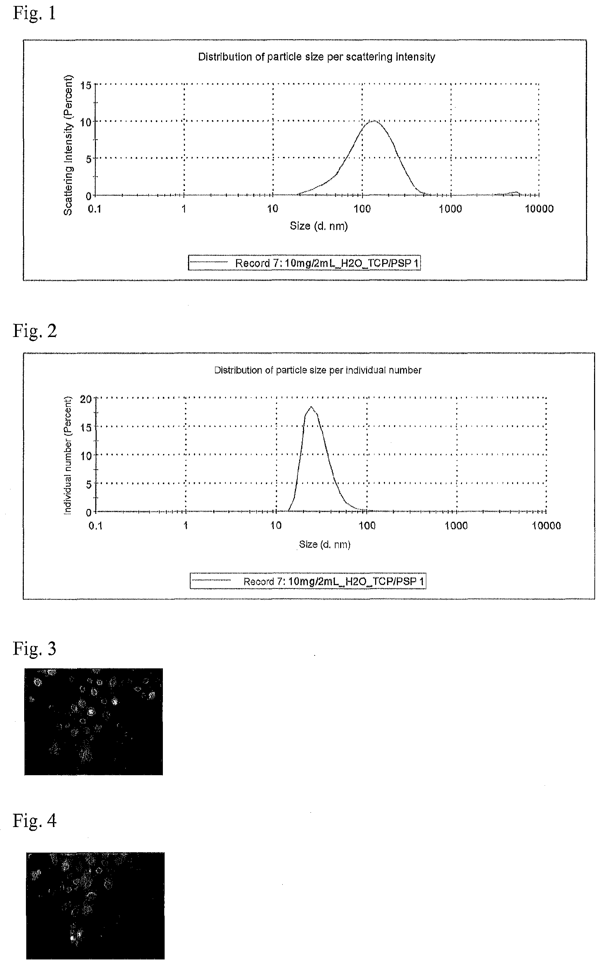

Some of the calcium salt compositions obtained in Preparation Example 1 were dispersed in water so that the calcium salt concentration would be 1.0 mg/2mL in each of them. The obtained dispersed solution was subjected for the measurement of particle size and zeta potential using DLS (manufactured by Malvern Instruments Ltd, Zetasizer Nano ZS). The results of those whose fitting analysis was well performed were shown in Table 4. Furthermore, in Example 6, the distribution of particle size per scattering intensity of DLS and the distribution of particle size per individual number of DLS were each shown in Fig. 1 and Fig. 2. Based on the results of these particle size and zeta potential, it was shown that the calcium salt in the dispersed solution are calcium salt microparticles having a particle diameter of several dozen nm to several hundred nm, and based on the zeta potential analysis, it was revealed that negatively charged particles are dispersed in water. Furthermore, by comparing Examples 19 and 20, it was shown that the particle size of the calcium salt microparticles would become smaller due to the increase of the amount of the dispersant at the time of grinding and mixing. In addition, when the results between Examples 6 and 7, and 27 to 30 are compared, it was revealed that the particle size of the calcium salt microparticles would become smaller by mixing two kinds of dispersants rather than that of the dispersant alone.

Examples 3 and 5 use polydisperse polysaccharides *per se* as a dispersant, and they were inadequate for the fitting analysis of the particle size in the present test.

**[Table 4]**

| | Average particle size of individual number | Average particle size of scattering intensity | Zeta potential |
|---|---|---|---|
| Example 3¹⁾ | | | -55.1mV |
| Example 5¹⁾ | | | -38.4mV |
| Example 6¹⁾ | 30nm | 144nm | -39.1mV |
| Example 7¹⁾ | 21nm, 44nm | 113nm | -38.8mV |
| Example 11¹⁾ | 36nm | 149nm | - |
| Example 12 ¹⁾ | 18nm, 39nm | 123nm | - |
| Example 13¹⁾ | 82nm | 172nm | - |
| Example 14¹⁾ | 35nm, 100nm | 195nm | - |
| Example 19²⁾ | 40nm | 162nm | - |
| Example 20³⁾ | 15nm, 35nm | 128nm | - |
| Example 25 ⁴⁾ | - | 131nm | - |
| Example 26²⁾ | - | 129nm | - |
| Example 27¹⁾ | 32nm | 96nm | - |
| Example 28¹⁾ | 15nm,31nm | 91nm | - |
| Example 29¹⁾ | 15nm,31nm | 90nm | - |
| Example 30 ¹⁾ | 38nm | 96nm | - |

| | | | |
|---|---|---|---|
| ¹⁾ Concentration of calcium salt composition: Dispersed at a concentration of 10mg/2mL. ²⁾ Concentration of calcium salt composition: Dispersed at a concentration of 5mg/2mL. ³⁾ Concentration of calcium salt composition: Dispersed at a concentration of 20mg/2mL. ⁴⁾ Concentration of calcium salt composition: Dispersed at a concentration of 2mg/2mL. -; Not performed | | | |

### (3) Dependency of particle size on grinding and mixing time

The calcium salt compositions sampled in Preparation Example 2 were dispersed in water so that the calcium salt concentration would be 1.0 mg/2mL in each of them. The obtained dispersed solution was subjected for the measurement of particle size using DLS (manufactured by Malvern Instruments Ltd, Zetasizer Nano ZS). The results were shown in Table 5.

The particle size tended to converge to a constant value as the grinding and mixing time increased until the grinding and mixing time reached 7.0 hours.

**[Table 5]**

| Grinding and Mixing time (upper column)/Average particle size of scattering intensity (lower column) | | | | |
|---|---|---|---|---|
| 0.5 hours | 1.0 hour | 3.0 hours | 5.0 hours | 7.0 hours |
| 238nm | 234nm | 165nm | 148nm | 145nm |

As mentioned above, when the average particle size of scattering intensity is from 50 nm to 300 nm, the calcium salt of the present invention has a good dispersity.

Moreover, regarding the zeta potential, when it is from -60 mV to -30 mV, the calcium salt of the present invention has a good dispersity.

Based on Tables 3 to 5, it was shown that the calcium salt composition obtained by mixing various calcium salts that are insoluble or poorly soluble to water with a dispersant has a good dispersity.

### 3. Dispersity test (B) (Dispersity: Dispersion method dependency)

According to Table 6, the dispersant was dissolved in a dispersion medium at a concentration of 9 mg/2 mL, and an aqueous solution or PBS solution was obtained. In each obtained aqueous solution or PBS solution, a powder of calcium salt (tricalcium phosphate) of Preparation Example 3 was dispersed so that the calcium salt concentration would be 1 mg/2 mL, and thereby the dispersed solutions of Test Examples 1 to 4 were prepared.

Moreover, according to Table 6, 50 mg of tricalcium phosphate and 450 mg of dispersant were each weighed, which were then subjected to a dry grinding and mixing process (25Hz, 90 minutes) together in a ball mill to obtain a calcium salt composition. The obtained calcium salt composition was dispersed in a dispersion medium so that the calcium salt concentration would be 1 mg/2 mL, and thereby the dispersed solutions of Test Examples 5 to 8 were prepared.

The obtained dispersed solution was allowed to stand at room temperature for 1 hour after preparation and was observed. As a result of this, it was found that Test Examples 5 to 8 in which a calcium salt and a dispersant were processed by dry grinding together in a ball mill demonstrated a superior dispersity as compared to those of the Test Examples 1 to 4 in which only tricalcium phosphate was processed by dry grinding in a ball mill.

**[Table 6]**

| | Composition | | Dispersion medium |
|---|---|---|---|
| | Calcium salt | Dispersant | |
| Test Example 1 | Tricalcium phosphate | Sodium alginate | Water |
| Test Example 2 | Tricalcium phosphate | Sodium polyphosphate | Water |
| Test Example 3 | Tricalcium phosphate | Sodium alginate | PBS |
| Test Example 4 | Tricalcium phosphate | Sodium polyphosphate | PBS |
| Test Example 5 | Tricalcium phosphate | Sodium alginate | Water |
| Test Example 6 | Tricalcium phosphate | Sodium polyphosphate | Water |
| Test Example 7 | Tricalcium phosphate | Sodium alginate | PBS |
| Test Example 8 | Tricalcium phosphate | Sodium polyphosphate | PBS |

### 4. Dispersity Test (C) (A dispersity test on water/various kinds of alcohol mixed dispersion medium)

The calcium salt composition of the Examples shown in Table 7 was dispersed in water/various kinds of alcohol mixed dispersion medium so that concentration of the calcium salt would be 1.0 mg/2mL, and thereby a dispersed solution was prepared. The obtained dispersed solution was allowed to stand at room temperature for 1 hour after preparation and was observed. A dispersed solution in which a precipitation was not confirmed by visual inspection was assessed as "○" and a dispersed solution in which a precipitation was confirmed by visual inspection was assessed as "×". The results were shown in Table 7.

**[Table 7]**

| | Water/Ethanol (vol/vol) | | | | | | |
|---|---|---|---|---|---|---|---|
| | 10/0 | 9/1 | 8/2 | 7/3 | 6/4 | 5/5 | 4/6 |
| Example 5 | ○ | ○ | ○ | ○ | ○ | ○ | ○ |

| | Water/Glycerin (vol/vol) | | | | | | |
|---|---|---|---|---|---|---|---|
| | 10/0 | 9/1 | 8/2 | 7/3 | 6/4 | 5/5 | 4/6 |
| Example 3 | ○ | ○ | ○ | ○ | ○ | ○ | ○ |
| Example 5 | ○ | ○ | ○ | ○ | ○ | ○ | ○ |
| Example 6 | ○ | ○ | ○ | ○ | ○ | ○ | ○ |
| Example 7 | ○ | ○ | ○ | ○ | ○ | ○ | ○ |

### 5. Dispersity Test (D) (Ultrafiltration Test)

The calcium salt composition obtained in Example 7 was dispersed in water to 25 mg/5 mL, and the obtained dispersed solution was condensed to about 1 mL by centrifugation (manufactured by KUBOTA Corporation, Tabletop centrifuge 4000, 3000 rpm, 1 hour) using an ultrafiltration filter (manufactured by Millipore Corporation, Amicon Ultra-15 3K). To the obtained supernatant, 4 mL of distilled water was added to obtain a mixed solution. Then, this was subjected for DLS measurement together with the filtrate. The result was shown in Table 8. As shown in Table 8, microparticles deriving from a calcium salt composition was confirmed in the supernatant. This implies that a calcium salt composition can be condensed and purified, as well as that a dispersion medium can be replaced with another dispersion medium.

**[Table 8]**

| | Average particle size of scattering intensity | |
|---|---|---|
| | Before ultrafiltration | After ultrafiltration (Supernatant) |
| Example 7 | 111nm | 110nm |

### 6. Dispersity Test (E) (Calcium salt concentration dependency)

The calcium salt compositions obtained in Examples 6 and 7 were dispersed in water to a concentration shown in Table 9. The obtained dispersed solution was allowed to stand at room temperature for 1 hour after preparation and was observed. A dispersed solution in which a precipitation was not confirmed by visual inspection was assessed as "o" and a dispersed solution in which a precipitation was confirmed by visual inspection was assessed as "×". The results were shown in Table 9. Based on the results of Table 9, it was shown that the calcium salt compositions of Examples 6 and 7 in which a calcium salt and a dispersant were processed by dry grinding together in a ball mill can disperse tricalcium phosphate successfully at a high concentration (1.0 to 3.0 mg/mL) as compared to those of the Test Example 2 in which only tricalcium phosphate was processed by dry grinding in a ball mill.

**[Table 9]**

| Concentration of calcium salt composition in a dispersed solution (mg/mL) | 5 | 10 | 30 |
|---|---|---|---|
| Concentration of calcium salt in a dispersed solution (mg/mL) | 0.5 | 1.0 | 3.0 |
| Example 6 | ○ | ○ | ○ |
| Example 7 | ○ | ○ | ○ |

### 7. Dispersity Test (F) (Preservation stability of the dispersed solution)

The calcium salt compositions obtained in Examples 3, 6 and 7 were dispersed in water to 25 mg/5 mL, and the obtained dispersed solution was subjected for a stability test in a thermostat at 45°C. The assessment of stability was performed by visual inspection, and in case of Examples 6 and 7 by DLS. A dispersed solution in which a precipitation was not confirmed by visual inspection was assessed as "o" and a dispersed solution in which a precipitation was confirmed by visual inspection was assessed as "×". The results were shown in Table 10. The calcium salt dispersed solution of the present invention showed a good preservation stability even after 9 weeks.

**[Table 10]**

| | Immediately after preparation | After 1 day | After 1 week | After 3 weeks | After 4 weeks | After 5 weeks | After 9 weeks |
|---|---|---|---|---|---|---|---|
| Example 3 | ○ | ○ | ○ | ○ | ○ | ○¹⁾ | ○¹⁾ |
| | | | | | | | (-) |
| Example 6 | ○ | ○ | ○ | ○ | ○ | ○¹⁾ | ○¹⁾ |
| | (134) | (133) | (127) | (129) | (-) | (133) | (-) |
| Example 7 | ○ | ○ | ○ | ○ | ○ | ○ | ○ |
| | (111) | (109) | (110) | (113) | (-) | (112) | (-) |

| | | | | | | | |
|---|---|---|---|---|---|---|---|
| The numerical values inside the parentheses indicate the average particle size (nm) of scattering intensity measured by DLS. ¹⁾ A very small amount of precipitate is confirmed by visual inspection -: Not performed | | | | | | | |

### 8. Filaggrin production test (A)

### (1) Preparation of a calcium salt supplemented medium

Various calcium salts were dispersed in water so that the concentration calculated in terms of calcium ion would be 0.89 M, this obtained dispersed solution was then sterilized in an autoclave (at 120°C for 20 minutes), and thereby various calcium salt stock dispersed solutions were prepared.

Then, according to Table 11, each calcium salt stock aqueous dispersed solution was added to a medium (manufactured by KURABO INDUSTRIES LTD., HuMedia-KG2) while pipetting so that that a predetermined supplemented concentration calculated in terms of calcium ion would be obtained, and thereby a calcium salt supplemented medium was prepared. In this present test, a calcium salt subjected to a dry grinding and mixing process in a ball mill (25Hz, 90 minutes) was used.

### (2) Filaggrin production test

On a culture plate of 24-wells to which the same medium as used in the preparation of the above-mentioned calcium salt supplemented medium was added, human skin keratinocytes (manufactured by KURABO INDUSTRIES LTD., NHEK) were inoculated so that the cell density would be 3,800 cells/cm², and this was cultured for 3 days (37°C, 5%CO₂). Then, this medium was exchanged with the calcium salt supplemented medium prepared as mentioned above and incubated for overnight (37°C, 5% CO₂).

Whether filaggrin is produced by a calcium salt was examined using fluorescent immunostaining methods by Anti-Filaggrin/FLG, Rabbit-Poly, Alexa Fluor 488 (manufactured by BIS, hereinafter referred to as "Anti-FLG" as well). Moreover, for comparing the staining, DAPI (4',6-diamidino-2-phenylindole dihydrochloride, manufactured by Wako Pure Chemical Industries, Ltd) was used to stain the nucleus.

That is to say that the above-mentioned incubated cells for overnight were washed with PBS and fixed with 4% paraformaldehyde solution (manufactured by Wako Pure Chemical Industries, Ltd). The fixed cells were permeabilized with 0.1% Triton-X 100, incubated for overnight with Anti-FLG PBS solution (x 50) at 4°C, and then stained with a PBS solution of 100 nM DAPI. Note that the cells were washed with PBS after every operation.

For calculating the relative production amount of filaggrin, a fluorescent microscopic image of the above-mentioned stained cells was taken. The brightness of the image was quantified using Photoshop CS3, and this was normalized by dividing the obtained numerical value with cell numbers (Brightness/Cell number; L_{cell}). Moreover, the increase rate of brightness by adding various calcium salts in comparison with L_{cell} of the Comparative Test Example 2 in which calcium salt aqueous dispersed solution has not been added to the medium was calculated. The results were shown in Table 11.

**[Table 11]**

| | Calcium salt | Supplemented concentration calculated in terms of calcium ion (mM) | Brightness/Cell number (L_{cell}) | Increase rate of brightness (%) |
|---|---|---|---|---|
| Test Example 9 | Calcium hydrogen phosphate | 0.89 | 0.28 | 290 |
| Test Example 10 | Calcium hydrogen phosphate | 1.78 | 0.26 | 270 |
| Test Example 11 | Calcium pyrophosphate | 0.89 | 0.28 | 290 |
| Test Example 12 | Calcium pyrophosphate | 1.78 | 0.25 | 260 |
| Test Example 13 | Tricalcium phosphate | 0.89 | 0.18 | 190 |
| Test Example 14 | Tricalcium phosphate | 1.78 | 0.26 | 270 |
| Test Example 15 | Calcium dihydrogen phosphate | 0.89 | 0.17 | 180 |
| Test Example 16 | Calcium glycerophosphate | 1.78 | 0.19 | 200 |
| Comparative Test Example 1 | Calcium chloride | 1.78 | 0.12 | 130 |
| Comparative Test Example 2 | - | 0 | 0.095 | 100 |

As mentioned above in Table 11, an increase of brightness of fluorescence deriving from Anti-FLG was confirmed by addition of any of the calcium salts. The brightness under the fluorescent microscope measured by fluorescent immunostaining method reflects the relative filaggrin production amount, and thus, based on this test, it was shown that calcium salts promote the filaggrin production in human skin keratinocytes.

In particular, calcium hydrogen phosphate, calcium pyrophosphate, and tricalcium phosphate showed a relatively high filaggrin production as compared to that of calcium chloride, and it was revealed that filaggrin production was promoted well even at low concentration of calcium salts.

The above mentioned calcium hydrogen phosphate, calcium pyrophosphate, and tricalcium phosphate had a low solubility to water and they were hardly dissolved in the medium when visually inspected. Despite of this, these calcium salts do promote the production of filaggrin and the topical contact/action of them to the cells is assumed to be the reason for this.

In other words, it is assumed that these calcium salts do not act to cells as calcium ions dissolved in a medium, but they act as calcium salt *per se*, and thereby have improved the production of filaggrin in cells.

### 9. Filaggrin production test (B)

Filaggrin production test (A) showed that a calcium salt insoluble or poorly soluble to water promotes filaggrin production. Therefore, a filaggrin production test was performed by using a calcium salt composition comprising a calcium salt and a dispersant.

### (1) Preparation of a calcium salt supplemented medium

Calcium salt composition (tricalcium phosphate/sodium polyphosphate) or calcium chloride of Example 6 was dispersed in water so that the concentration calculated in terms of calcium ion would be 0.089 M (92 mg/mL), this dispersed solution was then sterilized in an autoclave (120°C for 20 minutes), and thereby a calcium salt stock dispersed solution was prepared.

Then, according to Table 12, the calcium salt stock aqueous dispersed solution was added to the same medium as used in filaggrin production test (A) while pipetting so that a predetermined supplemented concentration calculated in terms of calcium ion would be obtained, and thereby a calcium salt supplemented medium was prepared.

### (2) Filaggrin production test

On a culture plate of 24-wells to which the same medium as used in filaggrin production test (A) was added, human skin keratinocytes (manufactured by KURABO INDUSTRIES LTD., NHEK) were inoculated so that the cell density would be 3,800 cells/cm², and this was cultured for 3 days (37°C, 5%CO₂). Then, this medium was exchanged with the calcium salt supplemented medium prepared as mentioned above and incubated for a day or two (37°C, 5% CO₂). These incubated cells were stained as in the above-mentioned filaggrin production test (A), and L_{cell} and the increase rate of brightness were calculated. The results were shown in Table 12.

**[Table 12]**

| | Calcium salt | Supplemented concentration calculated in terms of calcium ion (mM) | Cells that were incubated for 1 day | | Cells that were incubated for 2 days | |
|---|---|---|---|---|---|---|
| | | | Brightness/Cell number (L_{cell}) | Increase rate of brightness (%) | Brightness/Cell number(L_{cell}) | Increase rate of brightness (%) |
| Test Example 17 | Example 6 | 0.09 | 0.22 | 90 | 0.23 | 130 |
| Test Example 18 | Example 6 | 0.27 | 0.27 | 110 | 0.19 | 100 |
| Test Example 19 | Example 6 | 0.89 | 0.37 | 150 | 0.86 | 480 |
| Comparative Test Example 3 | Calcium chloride | 0.09 | 0.20 | 83 | 0.16 | 89 |
| Comparative Test Example 4 | Calcium chloride | 0.27 | 0.19 | 79 | 0.12 | 67 |
| Comparative Test Example 5 | Calcium chloride | 0.89 | 0.23 | 96 | 0.20 | 110 |
| Comparative Test Example 6 | - | 0 | 0.24 | 100 | 0.18 | 100 |

Example 6 that is dispersible in water [Tricalcium phosphate/Sodium polyphosphate: 1/9(wt/wt)] was used in performing a filaggrin production test. As a result of this, it was shown that by the addition of a calcium salt composition to a medium as in Test Examples 13 and 14, filaggrin production was promoted. As the brightness of the fluorescence microscope reflects the relative amount of filaggrin, the magnitude of the numerical value of the brightness *per se* does not have much significance. Nonetheless, in comparison to the Comparative Test Examples, the calcium salt composition of the present invention clearly showed that the filaggrin production was well promoted.

### 10. Filaggrin production test (C)

Filaggrin production test (B) showed the effectiveness of a calcium salt composition. The calcium salt composition of the present invention includes a calcium component and a dispersant component. Here, a filaggrin production test was performed to examine the influence of the various constituent components on filaggrin production. The calcium salt composition ground in Example 28 were used.

### (1) Preparation of test medium

The calcium salt composition of Example 28 was dispersed in water so that the concentration calculated in terms of calcium ion would be 19.3 mM (Example 28 Composition; 20 mg/mL), and a calcium chloride aqueous solution (2.15 mg/mL), sodium L-ascorbic acid phosphate aqueous solution (2.0 mg/mL), and sodium polyphosphate aqueous solution (16 mg/mL) were prepared. Then, these solutions were sterilized by using a sterilization filter (0.25 µm), and thereby stock aqueous dispersed solutions for testing were prepared.

Then, according to Table 13, the stock aqueous dispersed solution for testing was added to the same medium as used in filaggrin production test (A) while pipetting so that a predetermined supplemented concentration of each component would be obtained, and thereby a test medium was prepared.

**[Table 13]**

| Final supplemented concentration | | | |
|---|---|---|---|
| | Final supplemented concentration | | |
| | Calculated in terms of calcium ion | Sodium polyphosphate | L-Ascorbic Acid-2-Phosphate Sodium |
| Comparative Test Example 7 | 0.0 | 0.0 | 0.0 |
| Comparative Test Example 8 | 0.39mM | 0.0 | 0.0 |
| Comparative Test Example 9 | 0.0 | 0.32mg/mL | 0.0 |
| Comparative Test Example 10 | 0.0 | 0.0 | 0.04mg/mL |
| Test Example 20 | 0.39mM | 0.32mg/mL | 0.04mg/mL |

### (2) Filaggrin production test

On a culture plate of 24-wells to which the same medium as used in filaggrin production test (A) was added, human skin keratinocytes (manufactured by KURABO INDUSTRIES LTD., NHEK) were inoculated so that the cell density would be 3,800 cells/cm², and this was cultured for 3 days (37°C, 5%CO₂). Then, this medium was exchanged with a calcium salt supplemented medium prepared as mentioned above and incubated for 2 days (37°C, 5% CO₂). These incubated cells were stained as in the above-mentioned filaggrin production test (A), and observed under the microscope.

The results were shown in Figs. 3 to 7. As a result of this, in comparison to an unsupplemented medium (Comparative Test Example 7) and test media consisting of each of the components (Comparative Test Examples 8 to 10), a test medium supplemented with the calcium salt composition of Example 28 (Test Example 20) was confirmed to have the most clear green luminescence deriving from filaggrin. Based on this, it was shown that a calcium salt composition produced filaggrin well.

### 10. Filaggrin production test (D)

In filaggrin production tests (A) to (C), a calcium salt composition or an insoluble calcium salt was shown to be effective on the production of filaggrin by using fluorescent immunostaining methods. However, fluorescent immunostaining methods are methods observing only a part of cells adhering to a dish, and thus, it is rather difficult to be said to be quantitative. For this reason, a filaggrin production test was performed by using dot plot. Dot plot is a semi-quantitative method in which all of the cells adhering to a dish are collected, transferred to a membrane, and then immunologically stained. The calcium salt composition ground in Example 28 were used.

### (1) Preparation of calcium salt test medium

The calcium salt composition of Example 28 (Test Example 21) and calcium chloride (Comparative Test Example 12) was added to a medium so that the concentration calculated in terms of calcium ion would be 0.39 mM (manufactured by KURABO INDUSTRIES LTD., HuMedia-KG2), and thereby a calcium salt supplemented medium was prepared for each. Furthermore, an unsupplemented medium was also prepared (Comparative Test Example 11). Then, these media were sterilized by using a sterilization filter (0.25 µm), and thereby a medium for testing was prepared.

### (2) Filaggrin production test

On a culture plate of 24-wells to which the same medium as used in filaggrin production test (A), human skin keratinocytes (manufactured by KURABO INDUSTRIES LTD., NHEK) were inoculated so that the cell density would be 30x10⁴ cells/dish (φ100x21mm), and this was cultured for a day (37°C, 5%CO₂). Then, this medium was exchanged with a calcium salt supplemented medium prepared as mentioned above and incubated for 2 days (37°C, 5% CO₂).

Then, this was washed with PBS, and once the cells were detached using trypsin/EDTA (manufactured by KURABO INDUSTRIES LTD.), trypsin was neutralized with a trypsin neutralizing solution (manufactured by KURABO INDUSTRIES LTD.), and the cells were collected by centrifugation. The collected cells were substituted with PBS, centrifuged, and the supernatant was removed to prepare a cell pellet. The obtained cell pellet was lysed in a urea buffer and the supernatant was collected after centrifugation. The total protein mass contained in the obtained supernatant was estimated by using DC Protein Assay (manufactured by Bio-Rad Laboratories, Inc.). The results were shown in Table 14.

**[Table 14]**

| Total Protein Mass | |
|---|---|
| | The total protein mass (mg/mL) |
| Comparative Test Example 11 | 19.7 |
| Comparative Test Example 12 | 26.9 |
| Test Example 21 | 22.1 |

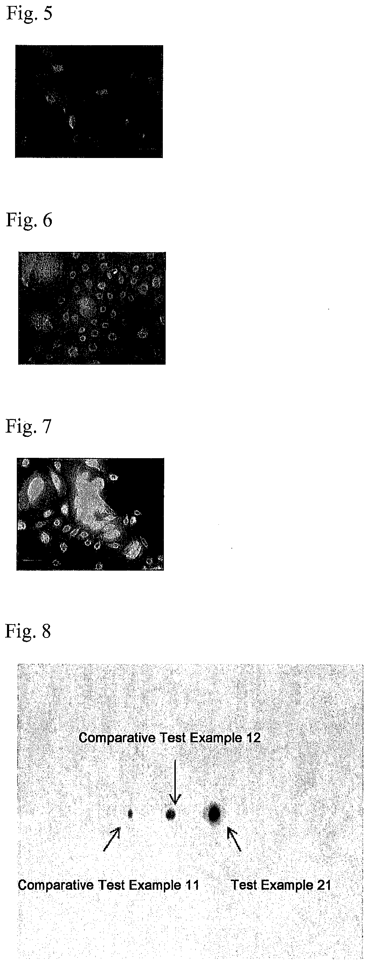

The obtained protein solution was transferred to a membrane (PVDF, manufactured by Millipore Corporation, Immobilon-P Transfer Membrane, 0.45 µm), blocked, and then incubated overnight at 4°C with a primary antibody (Anti-Filagrin(AKH1)(Mouse), Monoclonal, manufactured by Nacalai Tesque, Inc.). Then, this was washed with PBS-T, blocked, and then shaked for an hour with a secondary antibody (Polyclonal Goat, Anti-Mouse Immunoglobulins/HRP, manufactured by Dako Inc.). Then, this was washed with PBS-T and treated with ECL Prime Western Blotting Detection Reagent (manufactured by GE Healthcare). The image processing was carried out by C-DiGit (manufactured by LI-COR, Inc.). The results were shown in Fig. 8.

As shown in Fig. 8, as compared to Comparative Test Examples 11 and 12, in Test Example 21, a strong luminescent deriving from filaggrin protein was confirmed. Due to Table 14, as the total protein mass of Test Example 21, Comparative Test Examples 11 and 12 are of an amount of a same degree, it was shown that the calcium composition of the present invention produces filaggrin.

### INDUSTRIAL APPLICABILITY

The calcium salt composition of the present invention improves the filaggrin production promoting action of calcium salt, and thus, is useful as a filaggrin production promoter. Moreover, for this reason, the calcium salt composition of the present invention increases the skin barrier function, prevents the invasion of allergen, toxin, infectious organisms and the like, and is useful for the treatment and/or prevention of skin diseases such as allergic skin diseases including atopic dermatitis, contact dermatitis and urticarial.

Furthermore, the calcium salt composition of the present invention promotes the production of filaggrin caused by calcium salts and thereby increases the amount of amino acids which are the main component of natural moisturizing factors and the like. Accordingly, the calcium salt composition of the present invention is useful in the prevention, treatment and/or improvement of skin-aging symptoms such as formation of wrinkles, reduction of elasticity, rough skin, and reduction in moisture function in skin as an anti-aging agent.

Furthermore, the calcium salt composition of the present invention is useful as a whitening agent.

## Claims

1. A calcium salt composition comprising a calcium salt insoluble or poorly soluble to an aqueous medium and an anionic dispersant.

2. The calcium salt composition according to claim 1, further comprising an aqueous medium.

3. The calcium salt composition according to claim 1 or 2, wherein the weight ratio of the calcium salt and the dispersant is 1 : 0.5 to 1 : 100.

4. The calcium salt composition according to any one of claims 1 to 3, wherein the calcium salt is an organic salt or inorganic salt of calcium.

5. The calcium salt composition according to claim 4, wherein the organic salt of calcium is at least one selected from the group consisting of calcium glycerophosphate, calcium alginate, carboxymethylcellulose calcium, calcium citrate, calcium stearate, calcium myristate, and calcium 5'-ribonucleotides.

6. The calcium salt composition according to claim 4, wherein the inorganic salt of calcium is at least one selected from the group consisting of calcium hydrogen phosphate, calcium pyrophosphate, calcium dihydrogen pyrophosphate, tricalcium phosphate, calcium dihydrogen phosphate, calcium silicate, calcium carbonate, calcium oxide, calcium sulfate, and apatite.

7. The calcium salt composition according to any one of claims 1 to 6, wherein the anionic dispersant is at least one selected from the group consisting of ascorbic acid, tranexamic acid, water-soluble macromolecule, and phosphate derivatives.

8. The calcium salt composition according to claim 7, wherein the water-soluble macromolecule is at least one selected from the group consisting of gum arabic, tragacanth gum, galactan gum, guar gum, carrageenan, xanthan gum, deacylated gellan gum, maltose, trehalose, chitin, chitosan, agar, starch, dextrans, cyclodextrins, succinoglucan, collagen, casein, albumin, gelatin, hyaluronic acid and its salts, chondroitin sulfate, carboxymethylcellulose, methylhydroxypropyl starch, methylcellulose, pullulan, pectin, nitrocellulose, ethyl cellulose, methylhydroxypropyl cellulose, hydroxyethyl cellulose, sodium cellulose sulfate, hydroxypropyl cellulose, sodium carboxymethylcellulose, microcrystalline cellulose, sodium alginate, propylene glycol alginate ester, polyvinyl methyl ether, carboxyvinyl polymers, polyvinyl alcohol, polyvinyl pyrrolidone, polyoxyethylene polyoxypropylene copolymer macromolecules, acrylicpolymers, polyethylene imine, cationized guar gum, and cationized cellulose.

9. The calcium salt composition according to claim 7, wherein the phosphate derivative is at least one selected from the group consisting of phosphate, hydrogen phosphate, polyphosphate, metaphosphate, L-ascorbate-phosphate, adenosine-phosphate, dialkyl phosphate, monoalkyl phosphate, dipolyoxyethylene phosphate, monopolyoxyethylene phosphate, and phytate.

10. The calcium salt composition according to any one of claims 1 to 9 comprising at least two kinds of the anionic dispersant.

11. A filaggrin production promoter comprising the calcium salt composition according to any one of claims 1 to 10 or a calcium salt insoluble or poorly soluble to an aqueous medium.

12. An anti-aging agent comprising the calcium salt composition according to any one of claims 1 to 10 or a calcium salt insoluble or poorly soluble to an aqueous medium.

13. A filaggrin production promoter comprising a calcium salt insoluble or poorly soluble to an aqueous medium, which is ground by dry grinding.

14. A filaggrin production promoter comprising a calcium salt composition in which a calcium salt insoluble or poorly soluble to an aqueous medium and an anionic dispersant are dry ground together.

15. The filaggrin production promoter according to claim 13 comprising the calcium salt and an aqueous medium.

16. The filaggrin production promoter according to claim 14 comprising the calcium salt composition and an aqueous medium.

17. A method for producing a filaggrin production promoter comprising a step of dry grinding a calcium salt insoluble or poorly soluble to an aqueous medium.

18. The method for producing according to claim 17 further comprising a step of dispersing in an aqueous medium subsequent to the step of dry grinding.

19. A method for producing a filaggrin production promoter comprising a step of dry grinding a calcium salt insoluble or poorly soluble to an aqueous medium and an anionic dispersant together.

20. The method for producing according to claim 19 further comprising a step of dispersing in an aqueous medium subsequent to the step of dry grinding.

21. A method for promoting filaggrin production using the calcium salt composition according to any one of claims 1 to 10 or a calcium salt insoluble or poorly soluble to an aqueous medium.

22. A method for regulating the particle size of calcium salt **characterized in that** two or more kinds of the anionic dispersant are mixed with the calcium salt.
